Europäisches Patentamt

**European Patent Office** (11) Publication number: **0 039 999**

**Office européen des brevets** **A1**

(12) **EUROPEAN PATENT APPLICATION**

(21) Application number: **81301573.2** (51) Int. Cl.³: **A 61 M 5/14**
**A 61 K 31/21**

(22) Date of filing: **10.04.81**

(30) Priority: **09.05.80 US 148446**

(43) Date of publication of application:
**18.11.81 Bulletin 81/46**

(84) Designated Contracting States:
**AT BE CH DE FR GB IT LI LU NL SE**

(71) Applicant: **WARNER-LAMBERT COMPANY**
**201 Tabor Road**
**Morris Plains, New Jersey 07950(US)**

(72) Inventor: **Belsole, Susan Christine**
**RD 1, Box 207 C, South Road**
**Chester New Jersey 07930(US)**

(72) Inventor: **Goodhart, Frank William**
**22 Pepperigde Road**
**Morristown New Jersey 07960(US)**

(74) Representative: **Jones, Michael Raymond et al,**
**Haseltine Lake & Co. 28 Southampton Buildings**
**Chancery Lane**
**London WC2A 1AT(GB)**

(54) Intravenous system.

(57) A nitroglycerin infusion set suitable for the intravenous administration of a nitroglycerin solution to a patient by a catheter is disclosed, the set including a drip chamber for a solution, tube means for connecting the drip chamber to a catheter, and control means for controlling, in use, the rate of flow of the solution from said drip chamber through the tube means and catheter and into a blood vessel of a patient, wherein at least the inner surface of the tube means through which in use the flow of nitroglycerin solution is conducted is a polymeric material which is non-absorbent or substantially non-absorbent with respect to the nitroglycerin in solution. Preferably the inner surface of the tube means is a polymeric material comprising cross-linked polyethylene. Such a tube means eliminates substantially absorption of nitroglycerin, compared with the absorption occurring with many conventional tube means, and permits a physician to calculate more accurately the amount of nitroglycerine actually reaching the patient.

Croydon Printing Company Ltd

# INTRAVENOUS SYSTEM

This invention relates to a system suitable for the intravenous administration of a nitroglycerin solution in fluid form.

Nitroglycerin is a widely used coronary vasodilator which is administered by various routes for the management of angina pectoris. For acute attacks sublingual nitroglycerin tablets are commonly used and relief of pain usually follows in a matter of minutes. Intravenous administration of nitroglycerin in intravenous dextrose or saline solution has been utilized in hospital situations for the prophylactic management of angina or during open heart surgery. These intravenous nitroglycerin solutions have commonly been prepared extemporanteously in hospital pharmacies from sublingual nitroglycerin tablets with the result being a variable, non-standardized product.

The potency and stability of nitroglycerin intravenous solutions has been a matter of scientific inquiry. This inquiry was based on the suspicion, which was later followed by the confirmed observation, that nitroglycerin incorporated into certain commerical intravenous saline and dextrose solutions packaged in collapsible envelopes of polyvinyl chloride film had seemingly disappeared. The studies yielding these results demonstrated that the loss of nitroglycerin was due to absorption of the nitroglycerin by the plastics material of the collapsible plastics envelopes in which the intravenous solutions were packaged. A comprehensive review of the problem can be found in Amer. Jour. Hosp. Pharmacy, 36: 173-177 (February) 1979 and 37: 201-105 (February) 1980.

Intravenous solutions to which nitroglycerin has been added and which have been originally packaged in glass or polyolefin containers have been found to be quite acceptable with respect to nitroglycerin stability. Such packaging offers a convenient way to overcome one aspect of the problem.

However, there is an added variable which further complicates the problem of maintaining careful control during the intravenous administration of nitroglycerin. This variable stems from the fact that an appreciable length of flexible plastics tubing is normally employed for carrying the intravenous solutions being administered from the infusion bag or other container to a needle or catheter through which it is introduced into the vein of a patient. Absorption losses can also occur here too thus making the actual amount of nitroglycerin being administered highly uncertain. This is particularly true in the case of tubing and packaging materials comprising polyvinyl chloride. Although these polyvinyl chloride materials are highly desirable because of their relatively low cost, great flexibility and high degree of transparency, they exhibit a marked affinity for combining with or absorbing nitroglycerin. They combine so readily that they act to remove appreciable amounts of the nitroglycerin present from intravenous solutions passing through the tubing. At slow delivery rates, for example from 0.5 ml/min. to 2 ml/min., contact time with the tubing is prolonged and the resulting absorption quite significant. Thus, tests have shown that the nitroglycerin actually delivered may be only 20-30% of that estimated based on the volume delivered and on the initial concentration of nitroglycerin in the intravenous solution prepared for administration.

According to the present invention, there is provided a nitroglycerin infusion set suitable for the intravenous administration of nitroglycerin solution to a patient by a catheter, the set comprising a drip chamber for a solution, a tube means for connecting the drip chamber to a catheter, and control means for controlling, in use, the rate of flow of the solution from the drip chamber through the tube means and catheter and into a blood vessel of a patient, wherein at least the inner surface of the tube means through which in use the nitroglycerin solution is conducted is a polymeric material which is non-absorbent or substantially non-absorbent with respect to the nitroglycerin in solution.

More particularly, it has been found that the loss of nitroglycerin from solutions for intravenous infusion which are cannulated or catherized into the veins of a patient through plastic tubing may be substantially eliminated by utilizing tube means having internal surfaces of cross-linked polyethylene in contact with the intravenous nitroglycerin solution employed. Regardless of the flow rate and contact time of the solution with such cross-linked polyethylene it has been found that not less than 93% and as high as 100% of the nitroglycerin initially present in solution remains available and can actually be delivered even after contact with such polyethylene material for as long as three hours.

The use of infusion sets which incorporate tubing or other tube means where only cross-linked polyethylene comes into contact with the nitroglycerin solution enables the prescribing physician to be quite certain that the desired dose is being administered. Patients may be accurately monitored for therapeutic response and the obvious danger of inadvertent overdosing is eliminated. This may easily occur where intravenous nitroglycerin of a given concentration is prepared and while being administered the nitroglycerin is unknowingly being absorbed by contact with an absorbent tubing. Absorption may occur to the extent of 70 to 80% of the initially available nitroglycerin as noted in the scientific literature cited above. The attending physician on observing the limited therapeutic response attributable to the lower dosage level actually, but unknowingly, being administered to the patient may well act to achieve the desired therapeutic response by increasing the dose. Should the absorbent plastic tubing of the intravenous set employed become substantially saturated with nitroglycerin after being in use for some hours, a markedly increased dose would then be reaching the patient since less would be absorbed. The unexpectedly large dose of nitroglycerin thus introduced directly into the vein of a patient could have serious and possibly fatal consequences.

By providing infusion sets where the thin plastic tubing leading from the intravenous bottle or package is so formed that only cross-linked polyethylene or some other appropriate non-absorbent material comes into contact with the intravenous aqueous dextrose or saline with which the nitroglycerin is usually concurrently administered, these serious consequences are readily avoided and accurate monitoring of both the therapeutic dose and the therapeutic response can be achieved reliably and confidently.

Besides the thin plastic tubing which carries the intravenous solution to the patient, intravenous infusion sets normally comprise a clear transparent drip chamber through which the dropwise rate of infusion may be observed and monitored. The top of the drip chamber is usually provided with a sharp piercing pin for piercing the seal with which the package of intravenous solution being administered is provided. A hole or holes in the piercing pin allows or allow communication with the drip chamber, and from the drip chamber the intravenous solution passes into the thin plastic tubing provided. Flow control is accomplished by means of an adjustable pressure clamp mounted on the plastic tubing which can be adjusted to constrict the tubing as desired to allow an increased or decreased flow.

The piercing pin and fittings for the drip chamber are generally formed of a rigid, non-absorbing acrylonitrile-butadiene-styrene (ABS) copolymer. The transparent drip chamber itself is formed of a short length of a clear, flexible, transparent polymeric material to enable the drip rate to be readily observed. For this purpose however, a polyvinylchloride material can be used since the contact time is brief and the area of contact so limited that absorption is minimal.

The use in these infusion sets of tubing which is formed with a lining of cross-linked polyethylene or other suitable non-absorbing material and whose outer shell is a separate, other flexible, abrasion-resistant and transparent polymeric material has been found to be highly effective in minimizing nitroglycerin absorption. Particularly useful is the tubing formed by the coextrusion process disclosed and claimed in United States Patent No. 4,061,461. It has been found that use of intravenous infusion sets in which the tubing has an inner lining of cross-linked polyethylene and an outer shell of, for instance, cross-linked ethylene vinyl acetate copolymer provides a thoroughly reliable means for the infusion of accurate dosages of nitroglycerin in aqueous saline or dextrose solutions. Any absorption of the nitroglycerin is minimal so that titration of the patient with the desired dosage for optimum therapeutic response is readily achieved.

The cross-linked polyethylene lining material is preferably cross-linked by means of radiation as is well known in the art using levels of less than 100 megarads so that the molecular weight increases while still retaining its crystalline structure. No plasticizers are incorporated in the polyethylene and the preferred tubing, as described above, will retain its flexibility over a wide temperature range of from $-60^{\circ}$ to $+350^{\circ}F(-51^{\circ}$ to $+212^{\circ}C)$ although its use for infusion purposes does not not require exposure to such drastic conditions. Sterilization of the tubing and the assembled infusion set may be effected by either irradiation or by the use of ethylene oxide.

In order further to illustrate this invention the following Examples are given.

Example 1

A substantially saturated aqueous solution of nitroglycerin was prepared by adding 3.86 g of citric acid U.S.P. and 8.72 g sodium citrate, hydrous, to 900 ml of water for injection, U.S.P. at 45$^{O}$C. To this aqueous solution were added 50 ml of an alcoholic nitroglycerin concentrate which contained 1.64% NTG (2.5% excess). The resulting mixture was then brought to a volume of 1000 ml by adding the necessary amount of water for injection, U.S.P. The final solution obtained contained 0.8 mg/ml.

A sufficient amount of this aqueous solution of nitroglycerin, i.e. 10 ml, was added to 250 ml of a commercial solution of intravenous 5% dextrose in water U.S.P. so that a final solution containing about 30 mcg/ml was obtained.

Example 2

1.0 Millilitre aliquots of the nitroglycerin/ dextrose solution prepared in Example 1 were heat sealed inside respectively a series of separate lengths of extruded plastic tubing, the tubing length being chosen so that the exposed inner surface area was about 1.6 sq. cm. The several lengths of tubing were formed of (A) polyvinyl chloride, (B) an inner lining of ethylene vinyl acetate copolymer and an outer shell of polyvinyl chloride, (C) an inner lining of polyurethane and an outer shell of polyvinyl chloride, and (D) an inner lining of cross-linked polyethylene and an outer shell of cross-linked ethylene vinyl acetate.

The filled samples of tubing were placed in a mechanical shaker at room temperature and assayed hourly for nitroglycerin content. The following results were obtained. The percentages given represent the amount of nitroglycerin present after each period based on the original nitroglycerin concentration present being considered   as 100%.

CONTACT TIME

| TUBING | 1 hr. | 3 hr. | 7 hr. | 24 hr. | 48 hr. |
|--------|-------|-------|-------|--------|--------|
| A | 9.3 | 0 | 0 | 0 | 0 |
| B | Nil | 0 | 0 | 0 | 0 |
| C | Nil | 0 | 0 | 0 | 0 |
| D | 98.2 | 98.8 | 94.4 | 88.1 | 87.1 |

Example 3

10 Millilitres of the 0.8 mg/ml solution of nitroglycerin obtained as described in Example 1 were injected quickly into a glass bottle containing 250 ml of 5% Dextrose Injection U.S.P.  The solution was mixed well by shaking for one minute.  The solution obtained contained about 30 mcg/ml of nitroglycerin. 10 Millitres of the solution were withdrawn immediately, placed in a glass stoppered volumetric flask sealed with parafilm and then refrigerated until assayed.  The glass bottle with the remaining nitroglycerin solution was maintained at room temperature and 10 ml samples were withdrawn after a period of 24, 48, 72 and 96 hours, respectively, had elapsed.  The withdrawn samples were maintained in sealed and stoppered glass volumetric flasks under refrigeration until assayed.  When assayed for nitroglycerin content the samples showed that they had retained 100% of their original potency of nitroglycerin as first prepared. No loss was experienced in glass bottles.

An analogous stability test where a cross-linked polyolefin bottle was used as the storage container for the bulk material showed a similar result.

Example 4

10 Millilitres of the 0.8 mg/ml solution of nitroglycerin obtained as described in Example 1 were injected quickly into a glass bottle containing 250 ml of 5% Dextrose Injection U.S.P.  The solution was mixed well by shaking for one minute.  The solution obtained contained about 30 mcg/ml of nitroglycerin.  2 Millilitres of the resulting solution were withdrawn immediately after mixing and stored in a 5 ml glass volumetric flask stoppered and parafilm sealed under refrigeration until tested for nitroglycerin content.

The solution which remained was then attached to a commerically available intravenous infusion set (Burron AD-200, Lot 09J1315) consisting of an ABS piercing tube, a cylindrical PVC drip chamber and nylon filter, but with about 165 cm of "Bev-a-Line V" tubing (cross-linked polyethylene liner and ethylene vinyl acetate copolymer outer shell) being employed in place of the usual polyvinyl chloride tubing.  The flow rate through the tubing was adjusted with the flow control means to a rate of about 2 ml/minute.  Samples of 2 ml volume were collected at predetermined intervals with the initial collection starting after the first 5 minutes.  Additional 2 ml samples were taken starting at the 10 minute, 15 minute, 20 minute, 40 minute, 60 minute, 80 minute, 100 minute, 120 minute and 140 minute marks.  All samples were collected in 5 ml glass stoppered volumetric flasks sealed with parafilm parafilm and stored under refrigeration until tested for nitroglycerin content.  Taking the nitroglycerin content of the first sample drawn as 100% the following results were determined.

MINUTES

| CONTROL | 5 | 10 | 15 | 20 | 40 | 60 | 80 | 100 | 120 | 140 |
|---------|-----|-----|-----|-----|-----|-----|-----|-----|-----|-----|
| 100% | 100 | 100 | 100 | 100 | 100 | 100 | 100 | 100 | 100 | 100 |

By way of comparison, with the usual polyvinyl chloride tubing, the results observed using the above procedure were as follows:

MINUTES

| CONTROL | 10 | 20 | 40 | 60 | 80 | 100 | 120 | 150 |
|---------|------|------|------|------|------|-----|------|------|
| 100% | 60.7 | 61.4 | 59.3 | 78.6 | 79.3 | 80 | 91.7 | 90.3 |

CLAIMS:

1.    A nitroglycerin infusion set suitable for the intravenous administration of a nitroglycerin solution to a patient by a catheter, the set comprising a drip chamber for a solution, a tube means for connecting the drip chamber to a catheter, and control means for controlling, in use, the rate of flow of the solution from the drip chamber through the tube means and catheter and into a blood vessel of a patient, wherein at least the inner surface of the tube means through which in use the nitroglycerin solution is conducted is a polymeric material which is non-absorbent or substantially non-absorbent with respect to the nitroglycerin in solution.

2.    A nitroglycerin infusion set in accordance with claim 1, in which the inner surface of the tube means is a polymeric material comprising cross-linked polyethylene.

European Patent Office

**EUROPEAN SEARCH REPORT**

EP 81 30 1573.2

## DOCUMENTS CONSIDERED TO BE RELEVANT

| Category | Citation of document with indication, where appropriate, of relevant passages | Relevant to claim |
|---|---|---|
| P,X | JOURNAL DE PHARMACIE DE BELGIQUE, Vol. 35, May 1980 Brussels F. MATHOT et al. "Les perfusions de nitroglycérine. Etude de l'absorption par différents matériaux plastiques" pages 389 to 393 * complete document * | 1 |
| A | DE - B2 - 2 800 484 (TERUMO CORP.) * claim 1; column 2, lines 53 to 59 * | 2 |
| A | JOURNAL OF PHARMACEUTICAL SCIENCES, Vol. 66, No. 9, September 1977 Washington M.J. PIKAL et al. " Polymer Sorption of Nitroglycerin and Stability of Molded Nitroglycerin Tablets in Unit-Dose Packaging" pages 1293 to 1297 * abstract; page 1296, column 1, paragraph 2 * | |
| A | DE - A1 - 2 649 162 (A. NATTERMANN) * page 2, last sentence; page 3, last sentence * | |
| A | DE - A - 2 228 016 (UNION CARBIDE) * page 7, last paragraph * | |
| A | | |

**CLASSIFICATION OF THE APPLICATION (Int. Cl.³)**

A 61 M   5/14
A 61 K  31/21

**TECHNICAL FIELDS SEARCHED (Int. Cl.³)**

A 61 M   5/14
A 61 M   5/31
A 61 K  31/21
A 61 J   1/00
B 29 C  27/14
B 32 B  27/32

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| Berlin | 03-08-1981 | CLOT |

EPO Form 1503.1  06.78